# EUROPÄISCHE PATENTANMELDUNG

(11) **EP 1 820 560 A1**
(43) Veröffentlichungstag der Anmeldung: **22.08.2007**
(21) Anmeldenummer: 06003189.5
(22) Anmeldetag: 16.02.2006
(51) Int. Cl.: B01D 53/86, B01J 21/00, B01J 23/00, F22B 37/00, F22B 37/04, F22B 37/10

(54) **Dampferzeuger mit katalytischer Beschichtung von Wärmetauscheroberflächen zur Abgasreinigung**

(71) Anmelder: SIEMENS AKTIENGESELLSCHAFT, 80333 München (DE)
(72) Erfinder: Brückner, Jan, 91096 Möhrendorf (DE)

(57) **Zusammenfassung**

Ein Dampferzeuger (2, 2') einer technischen Anlage, insbesondere einer Kraftwerksanlage, mit einem von einer gasdichten Umfassungswand (8, 8') umschlossenen Heizgaskanal (20, 20'), wobei der Heizgaskanal (20, 20') eine Anzahl von von einem Strömungsmedium durchströmbaren Heizflächen aufweist, soll bei einem besonders geringen Konstruktions- und Herstellungsaufwand eine besonders zuverlässige Reinigung des aus dem Heizgaskanal (20, 20') abströmenden Heizgases gewährleisten. Dazu ist erfindungsgemäß vorgesehen, dass wenigstens eine der Heizflächen auf ihrer dem Heizgas zugewandten Seite zumindest teilweise mit einer katalytischen Beschichtung (44) versehen ist.

## Beschreibung

Die Erfindung betrifft einen Dampferzeuger einer technischen Anlage, insbesondere einer Kraftwerksanlage, mit einem von einer gasdichten Umfassungswand umschlossenen Heizgaskanal, wobei der Heizgaskanal eine Anzahl von einem Strömungsmedium durchströmbaren Heizflächen aufweist.

Bei einer Kraftwerksanlage mit einem Dampferzeuger wird das bei der Verbrennung eines fossilen Brennstoffes brennerseitig erzeugte Heizgas oder das aus einer Gasturbine abströmende heiße Abgas zur Verdampfung von einem Strömungsmedium im Dampferzeuger genutzt. Der Dampferzeuger weist zur Verdampfung des Strömungsmediums üblicherweise zu Heizflächen zusammengefasste oder gebündelte Dampferzeugerrohre auf, deren Beheizung durch die Strahlungswärme der Brennerflammen oder durch die konvektive Wechselwirkung mit dem Heizgas zu einer Verdampfung des darin geführten Strömungsmediums führt. Ein Teil der Heizflächen bildet dabei in der Regel direkt die gasdichte Umfassungswand des auch als Gaszug bezeichneten Heizgaskanals, während ein anderer Teil der Heizflächen zur Vergrößerung der effektiv nutzbaren Oberfläche in den Heizgaskanal eingehängt ist und in diesen hinein ragt. Der durch den Dampferzeuger bereitgestellte Dampf wiederum kann beispielsweise für einen angeschlossenen externen Prozess oder auch für den Antrieb einer Dampfturbine vorgesehen sein. Treibt der Dampf eine Dampfturbine an, so wird über die Turbinenwelle der Dampfturbine üblicherweise ein Generator oder eine Arbeitsmaschine betrieben. Im Falle eines Generators kann der durch den Generator erzeugte Strom zur Einspeisung in ein Verbund- und/oder Inselnetz vorgesehen sein.

Je nach Art des eingesetzten Brennstoffes und abhängig von den Auslegungscharakteristika des Dampferzeugers sind die den Dampferzeuger oder die ihm nachgeschaltete technische Anlage verlassenden Abgase mit Schadstoffen in Form von Stickoxiden, Kohlenstoffoxiden und/oder Schwefeloxiden sowie Festkörperpartikeln wie Flugasche und/oder Ruß belastet, welche die Umwelt schädigen können. In modernen Kraftwerken wird zunehmend versucht, die Schadstoffemission bereits durch so genannte Primärmaßnahmen, die vornehmlich die Brenner des Dampfkessels oder der ihm vorgeschalteten Gasturbine betreffen, und die zu optimierten, emissionsarmen Brennprozessen führen sollen, gering zu halten. In den Fällen, in denen derartige Maßnahmen nicht möglich oder mit kostspieligen Umrüstungsmaßnahmen verbunden sind oder nicht ausreichen, um die gesetzlich vorgeschriebenen Grenzwerte zu unterschreiten, sind so genannte Sekundärmaßnahmen erforderlich, um die im Rauchgas bzw. Abgas enthaltenen Schadstoffe zu filtern, abzuscheiden oder auf sonstige Weise unschädlich zu machen, z. B. durch chemische Umwandlung in weniger umweltschädliche oder besser handhabbare Reaktionsprodukte.

Insbesondere sind zur Verringerung der im Heizgas vorhandenen Stickoxide so genannte DeNOₓ-Katalysatorvorrichtungen gebräuchlich, die an geeigneter Stelle mittels zugehöriger Tragkonstruktionen in den Heizgaskanal des Dampferzeugers eingehängt sind, siehe zum Beispiel die EP 0 753 701 A1. In einer derartigen DeNOₓ-Katalysatorvorrichtung werden die im vorbeiströmenden Heizgas enthaltenen Stickstoffoxide unter Mitwirkung des katalytischen Materials durch das Einsprühen oder Eindüsen einer Ammoniaklösung reduziert, wobei als Reduktionsprodukte Wasser (H₂O) und elementarer Stickstoff (N₂) anfallen. Das Verfahren wird auch als Selektive Katalytische Reduktion (SCR) bezeichnet. Nachteilig ist bei dem geschilderten Konzept, dass die DeNOₓ-Katalysatorvorrichtung zusätzlichen Einbauraum im Inneren des Heizgaskanals sowie vergleichsweise aufwändige Aufhänge- und Befestigungskonstruktionen erfordert, wodurch sich der Gesamtaufwand für die Errichtung und Montage des Dampfkessels erhöht. Bestehende Altanlagen können infolge fehlenden Einbauraumes zudem häufig nur mit vergleichsweise hohem Aufwand nachgerüstet und ertüchtigt werden.

Der Erfindung liegt daher die Aufgabe zugrunde, einen Dampferzeuger der eingangs genannten Art anzugeben, der einen besonders geringen Konstruktions- und Herstellungsaufwand erfordert, und bei dem besonders zuverlässig eine Reinigung des Heizgases gewährleistet ist, bevor dieses den Dampferzeuger ausgangsseitig verlässt.

Diese Aufgabe wird erfindungsgemäß dadurch gelöst, dass wenigstens eine der Heizflächen auf ihrer dem Heizgas zugewandten Seite zumindest teilweise mit einer katalytischen Beschichtung versehen ist.

Die Erfindung geht von der Überlegung aus, dass ein für einen besonders geringen Herstellungs- und Montageaufwand ausgelegter Dampferzeuger eine besonders geringe Bauhöhe - oder bei liegender Ausführung des Dampfkessels eine besonders geringe Baulänge - aufweisen sollte, so dass der Materialbedarf und der Zeitaufwand für die Fertigstellung der Umfassungswände sowie gegebenenfalls die statischen Anforderungen an die zugehörige Stützkonstruktion minimiert werden. Eine besonders kompakte und einfache Bauweise lässt sich erreichen, indem die bislang als separate Komponenten mit vergleichsweise hohem Bedarf an Einbauraum vorgesehenen Abgasreinigungsvorrichtungen in die ohnehin bereits vorhandenen und zum Betrieb des Dampferzeugers unbedingt notwendigen Wärmeübertragungselemente, das heißt insbesondere in die aus den Dampferzeugerrohren gebildeten Heizelemente und Heizflächen, integriert werden. Eine besonders effektive und platzsparende Integration der Reinigungsfunktion für das Heizgas lässt sich bei gleichzeitig hoher Reinigungswirkung erreichen, indem die Heizflächen nicht nur zur Wärmeübertragung, sondern zusätzlich als Träger für eine katalytisch aktive Oberflächenbeschichtung genutzt werden. Dabei ist die Beschichtung zweckmäßigerweise derart gewählt, dass sie durch den Kontakt und die Wechselwirkung mit dem vorbeiströmenden Heizgas einen Abbau oder eine Umwandlung von in diesem mitgeführten Schadstoffen bewirkt oder zumindest fördert, ohne selbst dabei "verbraucht" zu werden oder sich abzunutzen (Katalysatorprinzip). Nach dem nunmehr vorgesehenen Konzept entfallen somit insbesondere die Tragkonstruktionen für die bislang üblichen separaten Katalysatorvorrichtungen.

Bei einer für die Praxis besonders wichtigen Anwendung dieses Konzeptes ist vorteilhafterweise die auf die Heizflächen des Dampferzeugers aufgebrachte Oberflächenbeschichtung derart beschaffen, dass sie in Anlehnung an das an sich bereits bekannte und bewährte Funktionsprinzip der Selektiven Katalytischen Reduktion (SCR), welches auch in den bislang üblichen DeNOₓ-Katalysatorvorrichtungen und Entstickungsanlagen zum Einsatz kommt, eine Umwandlung oder einen Abbau der im Heizgas vorhandenen Stickoxide und/oder Kohlenstoffoxide katalysiert oder bewirkt. Zu diesem Zweck ist weiterhin vorteilhafterweise eine Eindüsevorrichtung für ein Reduktionsmittel, insbesondere eine ammoniakhaltige Flüssigkeit, derart im Heizgaskanal des Dampferzeugers angeordnet, dass beim Betrieb des Dampferzeugers das infolge der Eindüsung mit dem Reduktionsmittel versetzte Heizgas die jeweilige katalytisch beschichtete Heizfläche anströmt.

Mit anderen Worten: Die katalytisch aktive Beschichtung auf der oder den Heizflächen bzw. auf der Oberfläche der die jeweilige Heizfläche bildenden Dampferzeugerrohre dient dazu, eine Reaktion zwischen dem in das Heizgas eingebrachten Reduktionsmittel und den Stickoxiden des Heizgases einzuleiten und/oder aufrecht zu erhalten. Bei dem SCR-Verfahren werden die Stickoxide mit Hilfe des üblicherweise mit Luft als Trägerstrom in den Gaszug oder den Heizgaskanal eingedüsten Reduktionsmittels durch die Anwesenheit und Mitwirkung des Katalysatormaterials zu Stickstoff und Wasser reduziert.

Der Stickoxid-Anfall im Dampferzeuger ist in der Regel abhängig von der Art des verbrannten fossilen Brennstoffs und von der Betriebsweise des Dampferzeugers. Um die gesetzlich vorgeschriebenen Grenzwerte möglichst bei sämtlichen Betriebszuständen einzuhalten, wird daher üblicherweise die einzudüsende Reduktionsmittelmenge in Abhängigkeit von dem eingesetzten fossilen Brennstoff und von den momentanen Betriebsparametern variiert.

Besonders effektive katalytische Beschichtungen lassen sich unter Rückgriff auf neuere Erkenntnisse der Nanostrukturforschung verwirklichen. Ein mit Hilfe der Nanotechnologie erreichbares Auslegungsziel ist dabei insbesondere, dass sich das Beschichtungsmaterial einfach und dauerhaft auf nahezu beliebige Oberflächenkonturen der Dampferzeugerheizflächen aufbringen lässt. Für die Katalyse des Stickoxidabbaus kommen als katalytische Materialien vor allem Titanoxid, Vanadiumpentoxid oder Wolframoxid zum Einsatz. Alternativ können Katalsatoren auf Zeolih-Basis verwendet werden. Hierbei sind als bevorzugte Materialien insbesondere Ammoniummordenit und H-Beta-Zeolith zu nennen. Schließlich ist es auch denkbar, dass in der Zukunft Katalysatorwerkstoffe gefunden bzw. entwickelt werden, die auch ohne Eindüsung eines Reduktionsmittels oder einer sonstigen chemischen Reagenz einen Abbau von heizgasgetragenen Schadstoffen (z.B. Stickoxide) einleiten oder fördern. In diesem Fall könnte auf die oben beschriebene Eindüsevorrichtung verzichtet werden.

Bei der oder den katalytisch beschichteten Heizflächen kann es sich um Teilbereiche der Umfassungswand des Heizgaskanals bildende Wandheizflächen handeln. Zusätzlich oder alternativ können in den Heizgaskanal hineinragende Heizflächen oder sonstige Einbauteile mit einer katalytischen Oberflächenbeschichtung der genannten Art versehen sein. Insbesondere bieten sich zu diesem Zweck so genannte Schottheizflächen an. Unter einer Schottheizfläche ist dabei eine Anzahl von für den Durchfluss des Strömungsmediums parallel geschalteten, in einen gemeinsamen Eintritts- und in einen gemeinsamen Austrittssammler mündenden Dampferzeugerrohren zu verstehen, wobei die Dampferzeugerrohre dicht nebeneinander in einer Ebene liegen und somit eine Anzahl von plattenartigen Heizflächen bilden, die innerhalb des Gaszuges aufgehängt sind. Alternativ kann die beschichtete Heizfläche auch eine so genannte Rohrbündelheizfläche sein, bei der die einzelnen Rohre im Gegensatz zu einer Schottheizfläche nicht durch Stege miteinander verbunden sind. Insbesondere bei der Einbeziehung derartiger innen liegender Heizflächen ist die insgesamt für die katalytische Beschichtung zur Verfügung stehende Oberfläche vergleichsweise groß. Dadurch können bislang nicht erreichbare Reduktionsraten für die vom Heizgas getragenen Schadstoffe erzielt und somit auch ausgesprochen niedrig liegende Schadstoffgrenzwerte zuverlässig und dauerhaft unterschritten werden, ohne dass dazu den Fertigungsaufwand heraufsetzende oder den energetischen Wirkungsgrad des Dampferzeugers beeinträchtigende Kompromisse bei dessen Auslegung - z. B. ungünstige Temperaturprofile, Inkaufnahme von Strömungsinstabilitäten, aufwändige Befeuerungskonzepte und Brennerkonfigurationen etc. - erforderlich wären.

Für eine besonders effektive Abgasreinigung sollten die katalytisch beschichteten Heizflächen in einem Bereich des Heizgaskanals angeordnet sein, der hinsichtlich der in ihm vorherrschenden üblichen Betriebstemperaturen einen hohen Wirkungsgrad des üblicherweise temperatursensitiven katalytischen Reinigungsverfahrens gewährleistet. Bei einer Heizgasentstickung nach den SCR-Prinzip ist die jeweilige katalytisch beschichtete Heizfläche daher vorteilhafterweise in einer Region angeordnet, in der das bei Nennlastbetrieb des Dampferzeugers vorbeiströmende Heizgas eine Temperatur aus dem Intervall von etwa 300 °C bis 400 °C besitzt.

Das hier erläuterte Konzept lässt sich auf Dampferzeuger von unterschiedlicher Bauart und Betriebsweise, z. B. mit liegendem oder stehendem Kessel sowie mit Naturumlauf, Zwangsumlauf oder Zwangsdurchlauf, und mit unterschiedlichen Befeuerungskonzepten, z. B. Wirbelschichtfeuerung oder Trockenstaubfeuerung, anwenden. Auch kann die strömungsmediumseitige Verschaltung der Heizflächen, die beispielsweise Verdampferheizflächen, Überhitzerheizflächen sowie zu einem Economizer oder Luftvorwärmer gehörige Heizflächen umfassen können, beinahe beliebig vorgegeben sein.

In einer besonders bevorzugten ersten Variante, die insbesondere auch für einen beispielsweise einer Gasturbine nachgeschalteten Abhitzedampferzeuger geeignet ist, umfasst der Dampferzeuger einen im Wesentlichen in waagrechter Richtung vom Heizgas bzw. vom Abgas der Gasturbine durchströmten, auch als Horizontalgaszug bezeichneten Heizgaskanal mit einer Anzahl von jeweils mit einer katalytischen Beschichtung versehenen Heizflächen. Dabei kann es sich insbesondere um Verdampferheizflächen oder Economizerheizflächen in einem Bereich des Dampferzeugers handeln, in dem das vorbeiströmende Heizgas üblicherweise Temperaturen von etwa 300 °C bis 400 °C aufweist.

In einer ebenfalls besonders vorteilhaften zweiten Variante, die insbesondere bei fossiler Befeuerung mittels in den Dampferzeuger eingebauter Brenner zweckmäßig ist, ist der Dampferzeuger mit einem stehenden Kessel in so genannter Zweizugbauart errichtet und umfasst einen während des Betriebes von unten nach oben vom Heizgas durchströmten ersten Vertikalgaszug, der heizgasseitig über einen Horizontalgaszug mit einem von oben nach unten durchströmten zweiten Vertikalgaszug verbunden ist. In diesem Fall ist die jeweilige katalytisch beschichtete Heizfläche vorzugsweise im Bereich des Horizontalgaszuges oder des zweiten Vertikalgaszuges oder auch in einem diesem heizgasseitig nachgeschalteten Abschnitt des Heizgaskanals angeordnet, wobei es sich je nach vorgesehener Betriebstemperatur des Katalysatormaterials vorzugsweise um eine Überhitzerheizfläche, um eine Economizerheizfläche oder auch um eine Luftvorwärmeheizfläche handeln kann.

Die mit der Erfindung erzielten Vorteile bestehen insbesondere darin, dass durch das Aufbringen einer katalytisch aktiven, auf die Abgasreinigung und Entstickung ausgerichteten Beschichtung auf die in einem Dampferzeuger vorhandenen Heizflächen die bislang übliche, separaten Einbauraum beanspruchende DeNOₓ-Katalysatorvorrichtung entfallen kann, wodurch eine besonders kompakte und kostengünstige Bauweise für den Dampferzeuger ermöglicht ist. Da die für eine Beschichtung zur Verfügung stehenden Heizflächen vergleichsweise groß sind, lassen sich auch bei gering gehaltenen Anforderungen an den oder die Brenner sehr gute Schadstoff-Reduktionsraten erzielen. Zudem können bestehende Kraftwerke bzw. Dampferzeuger, in denen bislang noch keine sekundärseitigen Schadstoffminderungsmaßnahmen getroffen wurden, relativ leicht nachgerüstet und an steigende Umweltanforderungen und verschärfte gesetzliche Grenzwerte angepasst werden, z. B. durch nachträgliches Aufbringen einer katalytisch aktiven Beschichtung auf die vorhandenen Heizelemente oder durch den Austausch von unbeschichteten Heizflächen gegen beschichtete.

Verschiedene Ausführungsbeispiele der Erfindung werden anhand einer Zeichnung näher erläutert. Darin zeigen:
- FIG 1: schematisch einen fossil befeuerten Dampferzeuger in Zweizugbauart in Seitenansicht,
- FIG 2: schematisch einen Abhitzedampferzeuger mit liegendem Dampfkessel in Seitenansicht, und
- FGI 3: eine Draufsicht auf eine aus Dampferzeugerrohren gebildete Heizfläche eines Dampferzeugers.

Gleiche Teile sind in allen Figuren mit denselben Bezugszeichen versehen.

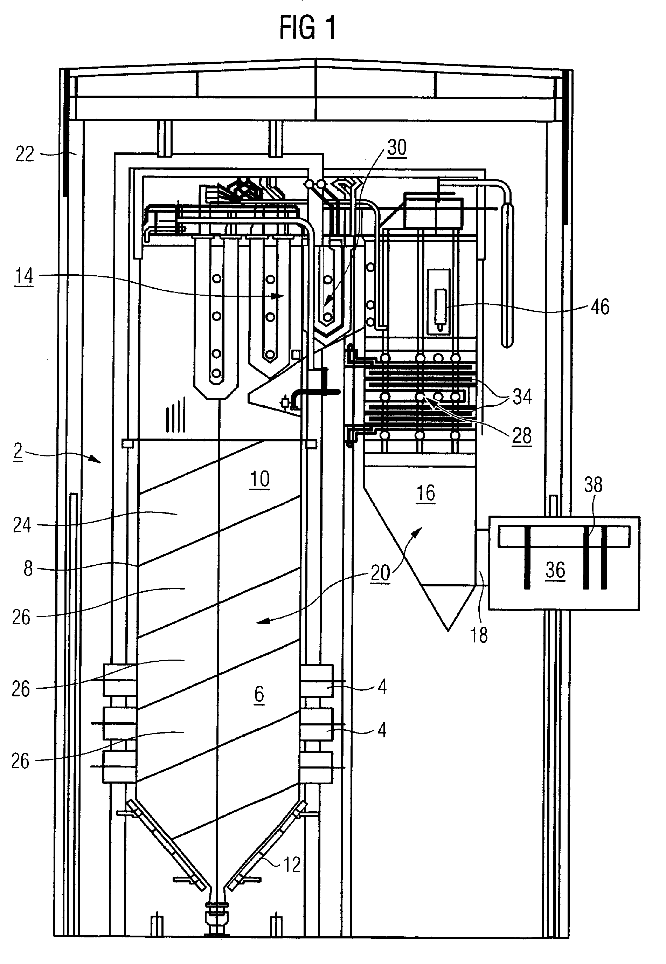

Der als Durchlaufdampferzeuger ausgelegte Dampferzeuger 2 gemäß FIG 1 umfasst eine Anzahl von Brennern 4 für einen fossilen Brennstoff. Die Brenner 4 sind in einer Brennkammer 6 angeordnet, die durch einen unteren Teil der Umfassungswand 8 eines ersten Vertikalgaszuges 10 gebildet ist. Die Umfassungswand 8 geht am unteren Ende des durch sie gebildeten ersten Verikalgaszuges 10 in einen trichterförmigen Boden 12 über. Der Dampferzeuger 2 ist in Zweizugbauart ausgeführt. Dazu ist dem ersten Vertikalgaszug 10 für aus der Verbrennung des fossilen Brennstoffes entstehendes Heizgas über einen Horizontalgaszug 14 ein zweiter Vertikalgaszug 16 nachgeschaltet. An den zweiten Vertikalgaszug 16 schließt sich ein weiterer horizontal verlaufender Gaszug 18 an, der schließlich in einen hier nicht gezeigten Kamin oder Schornstein mündet. Die Gaszüge 10,14,16,18 bilden in ihrer Gesamtheit einen Heizgaskanal 20. Die gesamte Anordnung ist vom Kamin abgesehen innerhalb einer Stützkonstruktion 22 angeordnet und über Verstrebungen durch diese abgestützt.

Die Umfassungswand 8 des ersten Vertikalgaszuges 10 ist aus nicht näher dargestellten Dampferzeugerrohren aufgebaut, die an ihren Längsseiten über Stege oder so genannte Flossen gasdicht miteinander verschweißt sind, und die sich im Wesentlichen schraubenförmig um den zylindrischen Innenraum 24 herum winden. Dabei ist jeweils eine Mehrzahl von zueinander benachbarten Dampferzeugerrohren für eine parallele Beaufschlagung mit Wasser als Strömungsmedium zu einer ein Segment der Umfassungswand 8 bildenden Verdampferheizfläche 26 zusammengefasst. Die Eintrittsenden der eine Verdampferheizfläche 26 bildenden Dampferzeugerrohre werden über einen (nicht dargestellten) gemeinsamen Eintrittssammler mit von einem Economizer 28 vorgewärmten Wasser beaufschlagt. Austrittsseitig strömt der in den Dampferzeugerrohren einer Verdampferheizfläche 26 infolge der Beheizung durch die Brenner erzeugte Wasserdampf über einen (nicht dargestellten) gemeinsamen Austrittssammler ab und wird anschließend einer Überhitzereinheit zugeführt.

Dazu ist den Verdampferheizflächen 26 des ersten Vertikalgaszuges 10 strömungsmediumssseitig eine Anzahl von als Schottheizflächen ausgeführten Überhitzerheizflächen 30 nachgeschaltet, die vornehmlich im Bereich des Horizontalgaszuges 14 angeordnet sind. Jede der überwiegend konvektiv beheizten Überhitzerheizflächen 30 umfasst eine Anzahl von für den Durchfluss des Strömungsmediums parallel geschalteten Dampferzeugerrohren. Die Dampferzeugerrohre einer Überhitzerheizfläche 30 sind miteinander zu einer Membranwand verbunden. Dazu ist jedes Dampferzeugerrohr der jeweiligen Überhitzerheizfläche 30 über jeweils einen Steg mit jedem ihm benachbarten Dampferzeugerrohr derselben Überhitzerheizfläche 30 verschweißt. Die jeweils einer Überhitzerheizfläche 30 zugeordneten Dampferzeugerrohre sind dabei dicht nebeneinander in einer Ebene liegend angeordnet und bilden damit als so genannte Schottheizfläche jeweils eine plattenartige Heizfläche. Die so gebildeten plattenartigen Überhitzerheizflächen 30 sind innerhalb des Horizontalgaszuges 14 aufgehängt. Der aus den Dampferzeugerrohren der Überhitzerheizflächen 30 abströmende, über die Verdampfungstemperatur hinaus überhitzte Dampf kann beispielsweise zum Antrieb einer hier nicht dargestellten Dampfturbine verwendet werden.

Ein Teil der Überhitzerheizflächen 30 kann auch zur Zwischenüberhitzung des aus einer ersten Turbinenstufe der Dampfturbine abströmenden, partiell entspannten Strömungsmediums eingesetzt werden, so dass das Strömungsmedium anschließend erneut aufgeheizt der nächsten Stufe der Dampfturbine zuführbar ist.

Infolge der Wärmeübertragung auf das die Verdampferheizflächen 26 und die Überhitzerheizflächen 30 durchströmende Strömungsmedium nimmt die Temperatur des im Heizgaskanal 20 strömenden Heizgases mit fortschreitendem Strömungsweg kontinuierlich ab. Beim Eintritt in den zweiten Vertikalgaszug 16 beträgt die Temperatur des Heizgases noch rund 300 °C bis 400 °C. Mit diesem Gehalt an Restwärme wird das durch die Rohre des auch als Speisewasservorwärmers bezeichneten Economizers 28 strömende, noch kalte und flüssige Strömungsmedium vor seinem Eintritt in die dem Economizer strömungsmediumsseitig nachgeschalteten Dampferzeugerrohre der Verdampferheizflächen 26 vorgewärmt. Durch eine derartige Verwertung der Heizgas-Abwärme lässt sich der Gesamtwirkungsgrad des Dampferzeugers um einige Prozentpunkte steigern. Der Economizer 28 umfasst mehrere jeweils aus strömungsmediumsseitig parallel geschalteten Rohren 32 gebildete Rohrbündelheizflächen, die Economizerheizflächen 34, die in den Heizgaskanal 20 hineinragen. Die Grundflächen der plattenartigen Economizerheizflächen 34 sind dabei parallel zur Strömungsrichtung des Heizgases ausgerichtet, so dass eine beidseitige Umströmung der Rohranordnung erfolgen kann. Die einzelnen Rohre 32 selbst sind bei der in FIG 1 dargestellten Ausführungsform senkrecht zur Strömungsrichtung des Heizgases angeordnet.

Nach dem Passieren der Economizerheizflächen 34 beträgt die Temperatur des Heizgases typischerweise nur noch rund 250 °C bis 400 °C, was jedoch für eine konvektive Beheizung des im Endbereich des Heizgaskanals 20 angeordneten Lufvorwärmers 36 ausreicht. Ähnlich wie der Economizer 28 weist der Luftvorwärmer 36 aus Rohrbündeln gebildete Heizflächen 38 auf, durch die jedoch nicht das zu verdampfende Strömungsmedium, sondern die den Brennern 4 des Dampferzeugers 2 zuzuführende Verbrennungsluft strömt, wodurch sie vor dem Eintritt in die Verbrennungszone vorgewärmt wird.

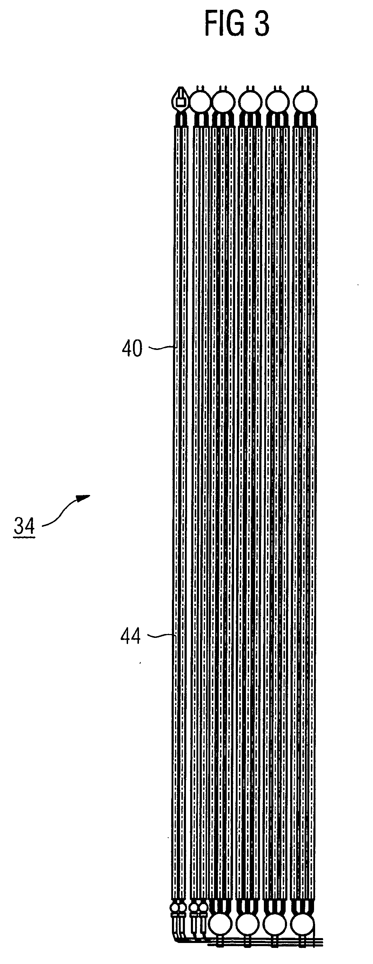

Der Dampferzeuger 2 ist bei kompakter und einfach gehaltener Bauweise für eine effektive Reinigung und Entstickung des aus dem Heizgaskanal 20 als Abgas abströmenden Heizgases ausgelegt. Zu diesem Zweck sind, wie in der in FIG 3 dargestellten Seitenansicht erkennbar ist, die Heizflächen 34 des Economizers 28, d. h. die das Strömungsmedium führenden Rohre 40, an ihrer dem Heizgas zugewandten Außenseite mit einer als Katalysator für die Einleitung und Aufrechterhaltung einer SCR-Entstickungsreaktion wirksamen Beschichtung 44 versehen. Als Beschichtungsmaterial kommt dabei z.B. Titanoxid oder ein Zeolithwerkstoff zum Einsatz, welches sich durch ein geeignetes, dem Fachmann geläufiges Beschichtungsverfahren vor der Montage des Dampferzeugers 2 auf das Grundmaterial der Rohre 40 und/oder der sie ggf. verbindenden Stege aufbringen lässt. Durch das Katalysatormaterial wird die für die SCR-Reaktion, bei der die im Heizgas mitgeführten Stickoxide durch eine in den Heizgasstrom eingedüste Ammoniaklösung zu elementarem Stickstoff und Wasser reduziert werden, erforderliche Aktivierungsenergie abgesenkt.

Die Ammoniakeindüsung erfolgt, wie in FIG 1 ersichtlich, mit Hilfe einer stromauf der Economizerheizflächen 34 im Heizgaskanal 20 angeordneten Eindüsevorrichtung 46, welche ihrerseits mittels einer (nicht dargestellten) Druckluftvorrichtung aus einem Vorratsbehälter für Ammoniak-Wasser bespeist wird. Die Düsen der Eindüsevorrichtung 46 sind derart eingestellt und ausgerichtet, dass eine möglichst gute Vermischung des ammoniakhaltigen Flüssigkeitsnebels mit dem Heizgas und eine möglichst gleichmäßige Benetzung der von dem entstehenden Gemisch angeströmten, katalytisch beschichteten Economizerheizflächen 34 erfolgt.

Bei einer weiteren, hier nicht gezeigten Ausführungsform sind anstelle der Economizerheizflächen 34 die Heizflächen 38 des Luftvorwärmers 36 mit der katalytischen Beschichtung versehen. In diesem Fall ist die Eindüsevorrichtung 46 in dem zwischen dem Economizer 28 und dem Luftvorwärmer 36 liegenden Abschnitt des Heizgaskanals 20 angeordnet. Je nach dem Betriebsbereich des Katalysatormaterials und dem beheizungsbedingt vorgegebenen Temperaturprofil entlang des Strömungsweges für das Heizgas kann es auch zweckmäßig sein, anstelle des Economizers 28 oder des Luftvorwärmers 36 die im Horizontalgaszug 14 angeordneten Überhitzerheizflächen 30 mit der katalytischen Beschichtung auszustatten.

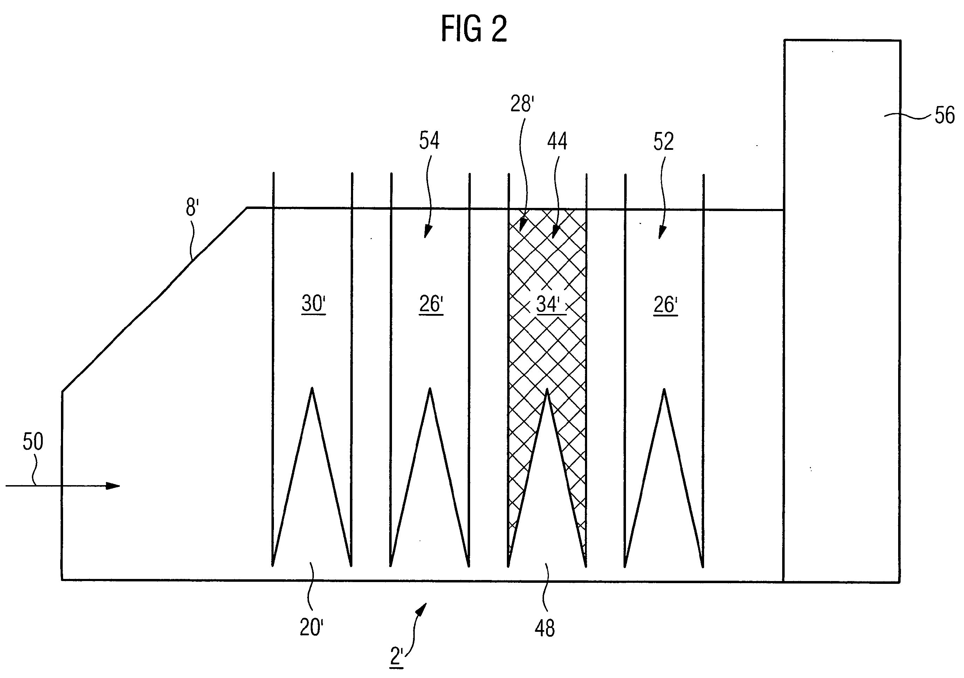

FIG 2 zeigt als weitere Ausführungsform einen als Abhitzedampferzeuger konzipierten Dampferzeuger 2' mit liegendem Wasserrohrkessel, der einer hier nicht dargestellten Gasturbine nachgeschaltet ist und dabei von dem aus der Gasturbine abströmenden Abgas beheizt wird. Das Abgas der Gasturbine durchströmt dabei den von der gasdichten Umfassungswand 8' umschlossenen Horizontalgaszug 48 in der mit dem Richtungspfeil 50 bezeichneten Richtung. Dabei gibt das Heizgas einen Großteil der in ihm enthaltenen Wärme durch konvektive Wärmeübertragung an die die Umfassungswand 8' bildenden Wandheizflächen oder an innerhalb des Heizgaskanals 20' angeordnete Rohrbündelheizflächen ab, wodurch das in den Heizflächen geführte Strömungsmedium vorgewärmt, verdampft und anschließend überhitzt wird. Dazu sind entsprechend strömungsmediumsseitig in Reihe geschaltete Economizerheizflächen 34', Verdampferheizflächen 26' und Überhitzerheizflächen 30' vorgesehen, wobei in dem in FIG 2 gezeigten Ausführungsbeispiel die Verdampferheizflächen 26' noch in die Heizflächen eines Mitteldruck-Verdampfers 52 und eines Hochdruck-Verdampfers 54 unterteilt sind. Das nach seiner Wärmeabgabe an das Strömungsmedium weitestgehend erkaltete Heizgas verlässt den Dampferzeuger 2' über einen als Vertikalgaszug ausgestalteten Kamin 56. Selbstverständlich sind dem Fachmann mannigfaltige Variationen hinsichtlich der Konfiguration und strömungsmediumsseitigen Verschaltung der Heizflächen geläufig, auf die es aber hier nicht im Einzelnen ankommt.

Entscheidend ist vielmehr, dass eine Anzahl von Heizflächen auf ihrer dem Heizgas zugewandten Seite zumindest teilweise mit einer katalytischen Oberflächenbeschichtung 44 versehen ist, welche einer Verringerung der im Heizgas mitgeführten Schadstoffe bewirkt oder fördert. Bei der Auswahl der zu beschichtenden Heizflächen ist auch hier wieder in Abhängigkeit von den für die katalytische Reaktion einzuhaltenden Temperaturgrenzen der örtliche Verlauf der Heizgastemperatur (im stationären Nennlastbetrieb) ein wichtiges Auslegungskriterium. Bei der in FIG 2 dargestellten Heizflächenkonfiguration kommen insbesondere die Heizflächen 26' des Mitteldruck-Verdampfers 52 und des Hochdruck-Verdampfers 54 sowie die Economizerheizflächen 34' in Betracht, wobei in FIG 2 beispielhaft die Economizerheizflächen 34' für die Beschichtung ausgewählt wurden. Die katalytische Beschichtung 44 ist in FIG 2 schematisch durch die Schraffur angedeutet. Ähnlich wie im Ausführungsbeispiel gemäß FIG 1 kann auch bei dem Dampferzeuger 2' nach FIG 2 wieder eine Eindüsevorrichtung für eine in das Heizgas einzudüsende chemische Reagenz stromauf der beschichteten Heizflächen im Heizgaskanal 20' angeordnet sein. Sie ist jedoch in FIG 2 der Übersichtlichkeit halber nicht eingezeichnet.

## Patentansprüche

1. Dampferzeuger (2, 2') einer technischen Anlage, insbesondere einer Kraftwerksanlage, mit einem von einer gasdichten Umfassungswand (8, 8') umschlossenen Heizgaskanal (20, 20'), wobei der Heizgaskanal (20, 20`) eine Anzahl von von einem Strömungsmedium durchströmbaren Heizflächen aufweist,
**dadurch gekennzeichnet, dass** wenigstens eine der Heizflächen auf ihrer dem Heizgas zugewandten Seite zumindest teilweise mit einer katalytischen Beschichtung (44) versehen ist.

2. Dampferzeuger (2, 2') nach Anspruch 1, bei dem die auf die jeweilige Heizfläche aufgebrachte Beschichtung (44) eine Umwandlung oder einen Abbau von im Heizgas vorhandenen Schadstoffen, insbesondere von Stickoxiden und/oder Kohlenstoffoxiden, katalysiert oder bewirkt.

3. Dampferzeuger (2, 2') nach Anspruch 1 oder 2 mit einer Eindüsevorrichtung (46) für ein Reduktionsmittel, insbesondere eine ammoniakhaltige Flüssigkeit, die derart angeordnet ist, dass beim Betrieb des Dampferzeugers (2, 2') das infolge der Eindüsung mit dem Reduktionsmittel versetzte Heizgas die jeweilige katalytisch beschichtete Heizfläche anströmt.

4. Dampferzeuger (2, 2') nach einem der Ansprüche 1 bis 3, wobei die katalytische Oberflächenbeschichtung (44) aus auf den Grundwerkstoff der jeweiligen Heizfläche aufbrachten Nanopartikeln aus Titanoxid und/oder Vanadiumpentoxid und/oder Wolframoxid und/oder aus einem Zeolithwerkstoff gebildet ist.

5. Dampferzeuger (2, 2') nach einem der Ansprüche 1 bis 4, wobei die jeweilige katalytisch beschichtete Heizfläche eine in die Umfassungswand (8, 8') integrierte Wandheizfläche ist.

6. Dampferzeuger (2, 2'), nach einem der Ansprüche 1 bis 5, wobei die jeweilige katalytisch beschichtete Heizfläche eine innerhalb des Heizgaskanals (20, 20') angeordnete oder in diesen hineinragende Heizfläche, insbesondere eine Schottheizfläche, ist.

7. Dampferzeuger (2, 2') nach einem der Ansprüche 1 bis 6, wobei die jeweilige katalytisch beschichtete Heizfläche in einem Bereich angeordnet ist, in dem das bei Nennlastbetrieb des Dampferzeugers (2, 2') vorbeiströmende Heizgas eine Temperatur aus dem Intervall von etwa 300 °C bis 400 °C besitzt.

8. Dampferzeuger (2') nach einem der Ansprüche 1 bis 7 in liegender Bauweise, insbesondere Abhitzedampferzeuger, mit einem im Wesentlichen in waagrechter Richtung vom Heizgas durchströmten Horizontalgaszug (48), bei dem die jeweilige katalytisch beschichtete Heizfläche eine Verdampferheizfläche (26') oder eine einem Economizer (28') zugeordnete Economizerheizfläche (34') ist.

9. Dampferzeuger (2) nach einem der Ansprüche 1 bis 7 in Zweizugbauart, insbesondere mit fossiler Befeuerung, mit einem während des Betriebs von unten nach oben vom Heizgas durchströmten ersten Vertikalgaszug (10), der heizgasseitig über einen Horizontalgaszug (14) mit einem von oben nach unten durchströmten zweiten Vertikalgaszug (16) verbunden ist, wobei die jeweilige katalytisch beschichtete Heizfläche eine im Bereich des Horizontalgaszuges (14) angeordnete Heizfläche, insbesondere eine Überhitzerheizfläche (30), oder eine im Bereich des zweiten Vertikalgaszuges (16) angeordnete Heizfläche, insbesondere eine Economizerheizfläche (34), ist.

10. Dampferzeuger (2, 2') nach einem der Ansprüche 1 bis 9, wobei die jeweilige katalytisch beschichtete Heizfläche Bestandteil eines auslassseitig im Heizgaskanal angeordneten Luftvorwärmers (36) ist.
